(19) Europäisches Patentamt
European Patent Office
Office européen des brevets

(11) Veröffentlichungsnummer: **0 253 257**
**B1**

(12) # EUROPÄISCHE PATENTSCHRIFT

(45) Veröffentlichungstag der Patentschrift: **14.11.90**

(21) Anmeldenummer: **87109694.7**

(22) Anmeldetag: **06.07.87**

(51) Int. Cl.⁵: **C07C 233/67**, C07C 311/16, C07D 213/82, C07C 217/78, A61K 31/165, A61K 31/18, A61K 31/44

(54) **Substituierte Amino -5,6,7,8-tetrahydronaphtyl-oxyessigsäuren, Verfahren zu deren Herstellung sowie die Verwendung als Arzneimittel.**

(30) Priorität: **16.07.86 DE 3623941**

(43) Veröffentlichungstag der Anmeldung: **20.01.88 Patentblatt 88/3**

(45) Bekanntmachung des Hinweises auf die Patenterteilung: **14.11.90 Patentblatt 90/46**

(84) Benannte Vertragsstaaten: **AT BE CH DE ES FR GB GR IT LI NL SE**

(56) Entgegenhaltungen:
**DE-C- 2 362 535**
**US-A- 3 362 988**

**CHEMICAL & PHARMACEUTICAL BULLETIN, Band 31, Nr. 7, 1983, Seiten 2329-2348, Verb. Nr. 22,39m,40m, Tokyo, JP; A. MIYAKE et al.: "Synthesis of 2-(N-substituted amino)-6-hydroxy-1,2,3,4-tetrahedronaphthalen-1-ol derivatives"**

(73) Patentinhaber: **BAYER AG, D-5090 Leverkusen 1 Bayerwerk(DE)**

(72) Erfinder: **Niewöhner, Ulrich, Dr., Gartenstrasse 3, D-5632 Wermelskirchen 3(DE)**
Erfinder: **Hoever, Franz-Peter, Dr., Kunstfelder Strasse 25, D-5000 Koeln 80(DE)**
Erfinder: **Junge, Bodo, Dr., Spiekern 23, D-5600 Wuppertal 23(DE)**
Erfinder: **Perzborn, Elisabeth, Dr., Am Tescherbusch 13, D-5600 Wuppertal(DE)**
Erfinder: **Seuter, Friedel, Dr., Moospfad 16, D-5600 Wuppertal 1(DE)**
Erfinder: **Fiedler, Volker-Bernd, Dr., Lehner Mühle 46, D-5090 Leverkusen 3(DE)**

**EP 0 253 257 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue Amino-5,6,7,8-tetrahydronaphthyl-oxyessigsäuren, Verfahren zu ihrer Herstellung sowie die Verwendung von Amino-5,6,7,8-tetrahydronaphthyl-oxyessigsäuren als Arzneimittel, insbesondere als Antithrombotika, Antiatherosklerotika und als antiischämische Mittel.

Thrombose und arteriosklerotische Gefäßveränderungen werden vor allem durch die Wechselwirkung zweier Metaboliten der Arachidonsäure, nämlich durch das Thromboxan $A_2$ ($TXA_2$) und durch das Prostacyclin ($PGI_2$) gesteuert. $TXA_2$ wirkt auf die Blutplättchen aggregierend, und $PGI_2$ hat eine antiaggregierende Wirkung. Darüber hinaus wirkt $TXA_2$ vasokonstriktorisch und $PGI_2$ vasodilatatorisch.

Bei einer Reihe von thrombo-embolischen und ischämischen Erkrankungen führt Hyperaggregabilität der Plättchen bzw. ein erhöhter Plättchenverbrauch zu einer gesteigerten Thromboxansynthese, so daß das $TXA_2$- und $PGI_2$-Gleichgewicht gestört ist. Es ist deshalb zur Therapie und Prophylaxe von thrombo-embolischen und ischämischen Erkrankungen wünschenswert, die Thromboxanwirkung zu hemmen und damit die protektive Eigenschaften des $PGI_2$ zu erhöhen.

Es wurde nunmehr überraschend gefunden, daß bestimmte Amino-5,6,7,8-tetrahydronaphthyl-oxyessigsäuren eine spezifische und starke antagonistische Wirkung bezüglich Thromboxan $A_2$ aufweisen.

Gefunden wurden Thromboxan-antagonistische und plättchenaggregationshemmende Amino-5,6,7,8-tetrahydronaphthyloxyessigsäuren der allgemeinen Formel (I)

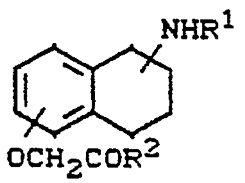

(I)

in welcher
$R^1$ für

für 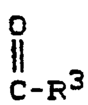

oder $SO_2R^4$ steht, wobei

$R^3$ für Aryl, substituiertes Aryl, Heteroaryl, Aralkyl oder die Gruppe

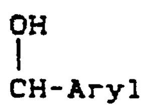

steht und wobei

$R^4$für Aryl oder substituiertes Aryl steht,

$R^2$für OH, Alkoxy, Phenoxy, Benzoxy oder $NR^5R^6$ steht wobei

$R^5$ und $R^6$ gleich oder verschieden sind und jeweils für Wasserstoff oder Alkyl stehen oder einer der Reste $R^5$ und $R^6$ für Benzyl steht,

sowei deren physiologisch verträglichen Salze mit ein- oder zweiwertigen Kationen.

Von besonderem Interesse sind Verbindungen der allgemeinen Formel (I) in welcher

$R^1$für CO-$R^3$ oder $SO_2R^4$ steht, wobei $R^3$ für Phenyl oder Naphthyl steht, welches gegebenenfalls 1, 2 oder 3 gleiche oder verschiedene Substituenten aus der Gruppe Halogen, Cyano, Trifluormethyl und Alkyl mit 1 bis 4 C-Atomen trägt oder für Pyridin, Chinolin oder Aralkyl mit 7 bis 12 Kohlenstoffatomen steht, wobei der Aralkylrest gegebenenfalls im Alkylteil durch Halogen oder Hydroxy substituiert ist und gegebenenfalls im Arylteil durch Halogen oder Alkyl mit 1 bis 4 Kohlenstoffatomen substituiert ist
oder für die Gruppe CHOH-Aryl steht wobei Aryl Phenyl oder Naphthyl bedeutet, welches gegebenenfalls 1 bis 3-fach substituiert ist durch Halogen, Cyano, Trifluormethyl oder Alkyl mit 1 bis 4 Kohlenstoffatomen,

$R^4$ für Phenyl oder Naphthyl steht, welche gegebenenfalls 1, 2 oder 3 gleiche oder verschiedene Substituenten aus der Gruppe Halogen, Cyano, Trifluormethyl und Alkyl mit 1 bis 4 C-Atomen tragen,

$R^2$ für Hydroxyl, Phenoxy, Benzoxy oder Alkoxy mit 1 bis 4 Kohlenstoffatomen steht oder für die Gruppe $NR^5R^6$ steht, wobei $R^5$ und $R^6$ gleich oder verschieden sind und jeweils für Wasserstoff oder Alkyl mit 1 bis 4 Kohlenstoffatomen stehen oder einer der Reste $R^5$ oder $R^6$ für Benzyl steht,

sowie deren physiologisch verträglichen Salze mit ein- oder zweiwertigen Kationen.

Von besonderem Interesse aus der Gruppe der Halogene sind Fluor und Chlor.

Die neuen Amino-5,6,7,8-tetrahydronaphthalin-oxyessigsäurederivate der Formel (I) können sowohl als Enantiomere, Enantiomerenpaare bzw. bei Vorliegen eines weiteren Asymetriezentrums in einem der Reste als Diastereomerenpaare vorliegen. Bevorzugt werden die optischen Isomere getrennt, indem man zunächst diastereomere Salze der Amine der Formel II mit optisch aktiven Säuren herstellt, diese diastereomeren Salze durch Kristallisation abtrennt und nachher die freien optisch aktiven Amine isoliert, oder indem man optisch aktive Imine der Ketone der Formel V herstellt mit (+) oder (-)α-Methylbenzylaminen, anschließend die Imindoppelbindung hydriert und in einem weiteren Hydrationsschritt den Benzylrest entfernt.

Es wurde ferner gefunden, daß man die Amino -5,6,7,8-tetrahydronaphthyl-oxyessigsäuren I erhält, wenn man die Amine der allgemeinen Formel (II)

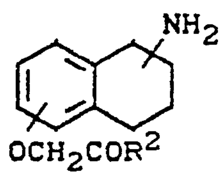

(II)

in denen $R^2$ die oben angegebene Bedeutung hat, in an sich bekannter Art und Weise mit Carbonsäuren der allgemeinen Formel $R^3$-COOH, wobei $R^3$ die oben angegebene Bedeutung besitzt, oder deren aktivierten Derivate wie Säurechloride, -anhydride oder aktivierte Ester oder mit Sulfonsäuren der allgemeinen Formel $R^4SO_3H$, wobei $R^4$ die oben angegebene Bedeutung besitzt, oder deren aktivierte Derivate wie Säurechloride oder aktivierte Ester umsetzt. Im Falle von $R^2$ OH schließt sich eine Verseifung zu den freien Carbonsäuren an.

Weiterhin wurde gefunden, daß man die Amino-5,6,7,8-tetrahydronaphthyl-oxyessigsäurederivate I auch erhält, wenn man die Phenole der allgemeinen Formel (III)

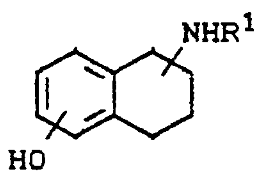

(III)

in denen $R^1$ die oben angegebene Bedeutung hat, mit Essigsäurederivaten der allgemeinen Formel (IV)

$X-CH_2-COR^2$(IV)

in denen

X eine Abgangsgruppe wie z.B. Cl, Br, J, $SO_2CH_3$,

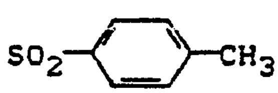

bedeutet und

$R^2$ die oben angegebene Bedeutung besitzt

in Gegenwart von säurebindenden Mitteln umsetzt.

Diese Verfahrensvariante ist besonders dann geeignet, wenn $R^1$ kein Schwefelatom enthält.

Im Falle von $R^2$ OH schließt sich eine Verseifung zu den freien Carbonsäuren an.

Die Aminotetralin-oxyessigsäurederivate II erhält man aus den entsprechenden Tetralonen V

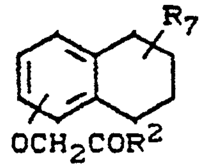

(V)

wobei

$R_7$ eine Ketogruppe ist und

$R^2$ die oben angegebene Bedeutung besitzt,

durch reduktive Aminierung analog literaturbekannter Verfahren (z.B. J. Am. Chem. Soc. 93, 2897 (1971); Rylander, "Catalytic Hydrogenation", S. 291-303, Academic Press, Inc., New York, 1967; Org.-Reactions 4, 174-255 (1948)).

Die Tetralone V erhält man durch Alkylierung der entsprechenden Hydroxytetraline VI

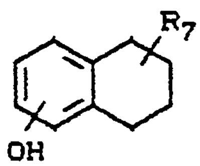

(VI)

in denen

$R_7$ die oben angegebene Bedeutung besitzt,

mit Essigsäurederivaten der allgemeinen Formel $X\text{-}CH_2\text{-}CO_2R^2$ (IV), in denen X und $R^2$ die oben angegebene Bedeutung haben, nach literaturbekannten Verfahren (z.B. Patai "The Chemistry of the Hydroxyl Group", pt. 1., S. 454-466, Interscience Publishers, New York, 1971; Tetrahedron 30, 1379 (1974)).

Die Hydroxytetralone VI sind teilweise literaturbekannt (z.B. J. Org. Chem. 14, S. 366 (1949)), teilweise können sie durch Etherspaltung analog literaturbekannter Verfahren aus den bekannten Methoxytetralonen XI hergestellt werden (z.B. Org. Syntheses, Vo, 51, S. 109; J. Chem. Soc. 1855 (1949)).

Die Aminotetralin-oxyessigsäurederivate II kann man ebenfalls erhalten, wenn man die Acetamide VII

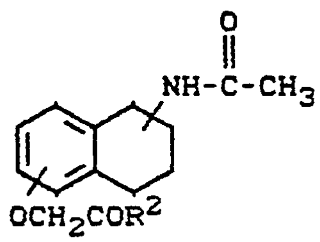

(VII)

in denen $R^2$ die oben angegebene Bedeutung hat, in bekannter Art und Weise sauer oder basisch hydrolysiert. VII läßt sich in analoger Weise wie bereits bei III → II beschrieben, durch Alkylierung der OH-Gruppe der Phenole VIII

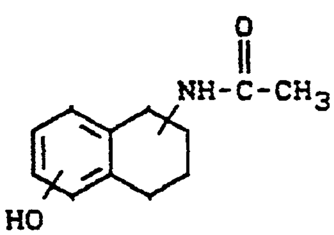

(VIII)

mit den Essigsäurederivaten IV erhalten. Die acetylierten Amino-hydroxytetraline VIII erhält man aus den entsprechenden Amino-hydroxytetralinen IX

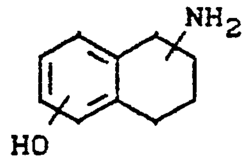

(IX)

bzw. deren Salzen mit anorganischen Säuren durch Acetylierung der Amino- und der Hydroxyfunktion und anschließender selektiver Verseifung des Esters nach allgemein bekannten Verfahren.

Aus den Aminohydroxytretralinen IX lassen sich analog nach literaturbekannten Verfahren (z.B. Chem. Ber. 103, 788 (1970) auch die Phenole der allgemeinen Formel III herstellen. Diese Vorgehensweise ist besonders geeignet, wenn $R^1$ kein Schwefelatom enthält. Die Phenole der allgemeinen Formel III lassen sich auch aus Verbindungen der allgemeinen Formel XII

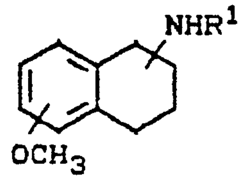

(XII)

durch Ätherspaltung nach bekannten Verfahren, beispielsweise durch Umsetzung mit $BBr_3$, herstellen.

Die Amino-hydroxy-tetralone IX sind teilweise bekannt (z.B. J. Med. Chem. 22, 1469 (1979)) oder können analog bekannter Verfahren über die Methoxy-aminotetraline X

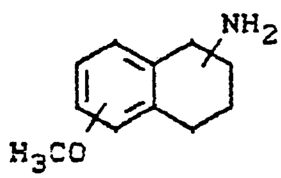

(X)

bzw. deren Salze mit anorganischen Säuren durch reduktive Aminierung aus den entsprechenden, bekannten Methoxytetralonen XI hergestellt werden,

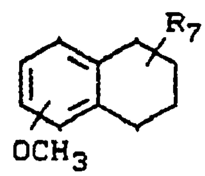

(XI)

in denen $R^7$ die oben angegebene Bedeutung besitzt.

Als Beispiel für die als Ausgangsstoffe verwendeten Methoxytretalone XI seien genannt:
5-Methoxy-1-tetralon
5-Methoxy-2-tetralon
6-Methoxy-1-tetralon
6-Methoxy-2-tetralon
6-Methoxy-3-tetralon

Die Synthesequenzen lassen sich ausgehend von den Methoxytetralonen XI in einem, Reaktionsschema wie folgt zusammenfassen:

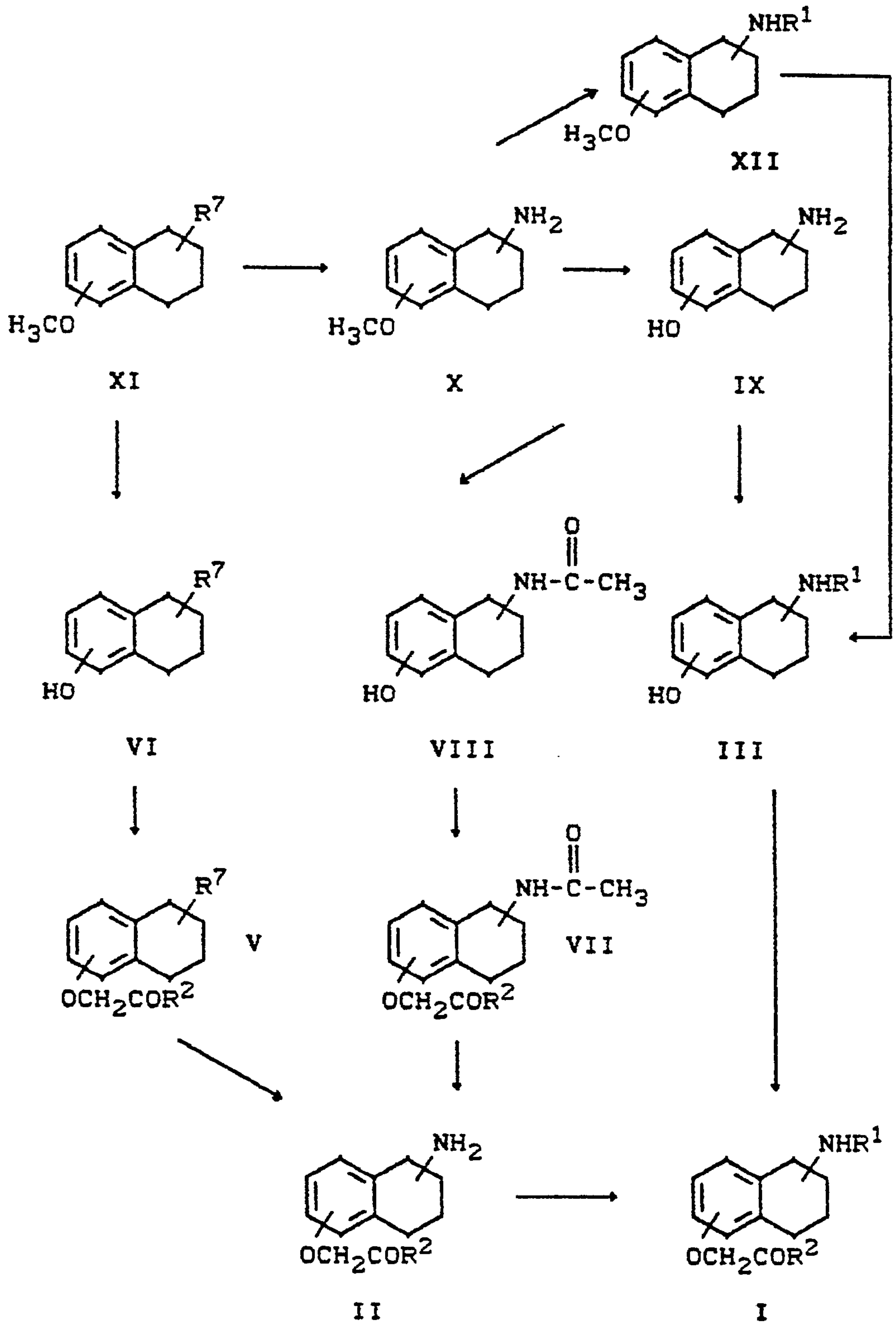

XII

XI

X

IX

VI

VIII

III

V

VII

II

I

wobei $R^1$, $R^2$, und $R^7$ die oben angegebenen Bedeutungen haben.

Im Falle von

$$R^1 = \overset{O}{\overset{\|}{C}}-R^3$$

wobei $R^3$ die oben angegebene Bedeutung besitzt, erhält man die Endprodukte I, indem man die Amine der allgemeinen Formel II mit den entsprechenden Carbonsäuren $R^2$-COOH oder deren aktivierte Derivate wie z.B. Säurechloride, Säureanhydride oder aktivierte Ester umsetzt.

Setzt man die aktivierten Derivate ein, so arbeitet man zweckmäßig in Gegenwart eines säurebindenden Mittels wie z.B. Alkali- oder Erdalkalihydroxide oder -carbonate oder organischen Basen wie Triethylamin, Pyridin oder N-Ethylmorpholin.

Als Lösungsmittel sind in Abhängigkeit von der Art des eingesetzten Carbonsäurederivate organische Lösungsmittel wie z.B. Methylenchlorid, Chloroform, Essigester, Tetrahydrofuran, Ether oder Dimethylformamid oder auch protische Lösungsmittel wie z.B. Wasser, Methanol, Ethanol geeignet.

Die Reaktionstemperatur liegt zwischen 0 und 100°C, vorzugsweise zwischen 0 und 30°C.

Setzt man die freien Carbonsäuren $R^3$-COOH ein, so kann die Umsetzung z.B. nach der in Chem. Ber. 103, 788 (1970) beschriebenen Art und Weise durchgeführt werden.

Im Falle von $E^1 = SO_2R^4$, wobei $R^4$ die oben angegebene Bedeutung besitzt, erhält man die Endprodukte I, indem man die Amine der allgemeinen Formel II mit den entsprechenden Sulfonsäuren $R^4SO_3H$ oder deren aktivierte Derivate wie z.B. Sulfonsäurechloride, Sulfonsäureanhydride oder Sulfonsäureester in Gegenwart eines säurebindenden Mittels wie z.B. Alkali- oder Erdalkalihydroxiden oder -carbonaten oder organischen Basen wie z.B. Triethylamin, Pyridin oder N-Ethylmorpholin umsetzt.

Asl Verdünnungsmittel sind die selben geeignet, die bereits für die Umsetzung mit den Carbonsäurederivaten genannt worden sind. Steht in den Verbindungen der allgemeinen Formel I $R^2$ für OAlkyl oder $NR^5R^6$, so kann sich eine Hydrolyse unter basischen oder sauren Bedingungen nach allgemein bekannter Art und Weise anschließen.

Man erhält die Verbindungen der allgemeinen Formel I auch nach der verfahrengemäßen Variante aus den Phenolen III, in denen $R^1$ die oben angegebene Bedeutung besitzt, indem man die Phenole III mit Essigsäurederivaten der allgemeinen Formel IV, in denen $R^2$ und X die oben angegebene Bedeutung besitzt, alkyliert.

Die Alkylierung wird zweckmäßig in Gegenwart eines säurebindenden Mittels wie z.B. Alkali- oder Erdalkalihydroxiden oder -carbonaten oder organischen Basen wie z.B. Triethylamin, Pyridin, Diazabicycloundecan in organischen Lösungsmitteln wie Aceton, Butanon, Dimethylformamid, Dimethylsulfoxid, Ethanol, Dioxan oder Toluol durchgeführt.

Oft ist es günstig ein Alkalihalogenid wie z.B. Natrium- oder Kaliumiodid und ein wasserbindendes Mittel wie Molekuklarsieb 3Å zuzusetzen.

Die Reaktionstemperatur liegt in Abhängigkeit vom Lösungsmittel zwischen 50 und 150°C, vorzugsweise zwischen 50 und 80°C.

Als Beispiele für die Verbindungen der allgemeinen Formel I seien genannt:

5-(4-Fluorphenylsulfonylamino)-5,6,7,8-tetrahydro-naphth-1-yl-oxyessigsäure
5-Phenylsulfonylamino-5,6,7,8-tetrahydro-naphth-1-yl-oxyessigsäure
5-(4-Methylphenylsulfonylamino)-5,6,7,8-tetrahydro-naphth-1-yl-oxyessigsäure
5-(3,4-Dichlorbenzoylamino)-5,6,7,8-tetrahydro-naphth-1-yl-oxyessigsäure
5-(3,4-Dichlorbenzoylamino)-5,6,7,8-tetrahydro-naphth-2-yl-oxyessigsäure
5-(4-Fluorphenylsulfonylamino)-5,6,7,8-tetrahydro-naphth-2-yl-oxyessigsäure
5-Phenylsulfonylamino-5,6,7,8-tetrahydro-naphth-2-yl-oxyessigsäure
6-(3,4-Dichlorbenzoylamino)-5,6,7,8-tetrahydro-naphth-1-yl-oxyessigsäure
6-(4-Fluorphenylsulfonylamino)-5,6,7,8-tetrahydro-naphth-1-yl-oxyessigsäure
6-(4-Chlorphenylsulfonylamino)-5,6,7,8-tetrahydro-naphth-2-yl-oxyessigsäure
6-(4-Fluorphenylsulfonylamino)-5,6,7,8-tetrahydro-naphth-2-yl-oxyessigsäure
7-(4-Fluorphenylsulfonylamino)-5,6,7,8-tetrahydro-naphth-2-yl-oxyessigsäure
7-(4-Chlorphenylsulfonylamino)-5,6,7,8-tetrahydro-naphth-2-yl-oxyessigsäure
7-(4-chlorphenylsulfonylamino)-5,6,7,8-tetrahydro-naphth-1-yl-oxyessigsäure
5-(4-Methylphenylsulfonylamino)-5,6,7,8-tetrahydro-naphth-2-yl-oxyessigsäure
6-(4-Methylsulfonylamino)-5,6,7,8-tetrahydro-naphth-2-yl-oxyessigsäure
5-(4-Trifluormethylphenylsulfonylamino)-5,6,7,8-tetrahydro-naphth-2-yl-oxessigsäure
5-(4-Cyanophenylsulfonylamino)-5,6,7,8-tetyrahydro-naphth-2-yl-oxyessigsäure
6-(4-Trifluormethylphenylsulfonylamino)-5,6,7,8-tetrahydro-naphth-2-yl-oxyessigsäure
6-(4-Cyanophenylsulfonylamino)-5,6,7,8-tetrahydro-naphth-2-yl-oxyessigsäure
5-(4-Cyanophenylsulfonylamino)-5,6,7,8-tetrahydro-naphth-1-yl-oxyessigsäure
5-(4-Trifluormethylphenylsulfonylamino)-5,6,7,8-tetrahydro-naphth-1-yl-oxyessigsäure
6-(4-Cyanophenylsulfonylamino)-5,6,7,8-tetrahydro-naphth-1-yl-oxyessigsäure
6-(4-Trifluormethylphenylsulfonylamino)-5,6,7,8-tetrahydro-naphth-1-yl-oxyessigsäure
6-(4-Methylphenylsulfonylamino)-5,6,7,8-tetrahydro-naphth-1-yl-oxyessigsäure

Als Zubereitungsformen kommen die üblichen galenischen Applikationsformen in Frage, beispielsweise Cremes, Tabletten, Pillen, Kapseln, Suppositorien, Emulsionen, Infusions- und Injektionslösungen. Diese Zubereitungsformen werden nach an sich bekannten Methoden hergestellt unter Verwendung üblicher Hilfs- und Trägerstoffe.

Der Einsatz der so hergestellten Arzneimittel erfolgt je nach Bedarf z.B. durch lokale, parenterale oder orale Verabreichung.

Besonders geeignet sind Formulierungen, die die erfindungsgemäßen Verbindungen in Konzentrationen von etwa 0,1 bis 10 Gew.-% enthalten. Besonders bevorzugt sind wäßrige Lösungen, die gegebenenfalls auf einen pH-Wert von 6 bis 8 gepuffert sind.

Die Dosierung der substituierten Amino-5,6,7,8-tetrahydronaphthyl-oxyessgisäurederivate in den erfindungsgemäßen Arzneimitteln liegt vorzugsweise in einem Bereich von 0,05 bis 100 mg/kg, insbesondere von 0,1 bis 20 mg/kg Körpergewicht.

Die in den erfindungsgemäßen Arzneimitteln enthaltenen substituierten Amino-5,6,7,8-tetrahydronaphthyl-oxyessigsäuren sind als Thromboxanantagonisten und Plättchenaggregationshemmer zur Verhütung und Behandlung von Thrombosen, Thromboembolien, ischämischen Erkrankungen, als Antiasthmatika und also Antiallergika geeignet.

Methodik

Thrombozytenaggregationshemmung in vitro

Für die in vitro-Bestimmung der thrombozytenaggregationshemmenden Wirkung wird Blut von gesunden Spendern, die mindestens 14 Tage lang kein Medikament eingenommen hatten, verwendet. Das Blut wird in 3,8 %iger Natriumcitratlösung aufgenommen. Plättchenreiches Plasma (PRP) wird durch 20 Min. lange Zentrifugation bei 150 g bei Raumtemperatur gewonnen (Jürgens/Beller: Klinische Methoden der Blutgerinnungsanalyse; Thieme Verlag, Stuttgart 1959). Die Plättchenaggregation wird nach der turbidometrischen Methode (Born, G.V.R.: J. Physiol. 162, 67, 1962) im Aggregometer bei 37°C bestimmt. Hierzu wird PRP mit der Prüfsubstanz bei 37°C inkubiert und anschließend die Aggregation durch Zugabe einer Kollagensuspension ausgelöst. Für die in-vitro-Versuche wird die minimal effektive Wirkstoffkonzentration (MEK) angegeben, die in den entsprechenden PRP-Proben die Thrombozytenaggregation hemmt.

Thrombozytenaggregationshemmung ex vivo

Für die ex vivo-Untersuchungen wird den Tieren die Wirksubstanz in einer Tylosesuspension oral verabreicht. Nach 90 Minuten werden die Tiere entblutet und das PRP mittels Zentrifugation gewonnen. Die Messung der Aggregationshemmung erfolgt analog dem Verfahren, welches für die in vitro-Versuche beschrieben ist; jedoch ohne Vorinkubation der Proben.

In der Tabelle sind Ergebnisse der Kollagen induzierter Thrombozytenaggregation einiger Beispiele aufgeführt.

| Beispiel Nr. | Hemmung der Thrombozytenaggregation (in vitro) Grenzkonzentration [mg/l] |
|---|---|
| 54 | 0,3 - 0,1 |
| 55 | 3 - 1 |
| 56 | 0,3 - 0,1 |
| 57 | 0,3 - 0,1 |
| 58 | 0,1 - 0,03 |
| 63 | 10 - 3 |
| 66 | 10 - 3 |

Beispiel 1

5-Amino-1-methoxy-5,6,7,8-tetrahydro-naphthalin-hydrochlorid

50 mmol des 5-Methoxy-1-tetralon, 0,5 Mol $NH_4OAc$ und 35 mmol $NaBH_3CN$ werden 24 h in 150 ml abs. MeOH bei RT gerührt. Es wird mit konzentrierter HCl bis pH 2 angesäuert, eingedampft, in 50 ml $H_2O$ aufgenommen und 2-3 × mit Ether ausgeschüttelt. Eventuell auftretende Niederschläge werden abfiltriert und mit der $H_2O$ Phase vereinigt. Die $H_2O$ Phase wird mit festem KOH auf pH 10 gestellt, mit NaCl gesättigt und 2 × mit Essigester ausgeschüttelt. Nach Trocknen mit $Na_2SO_4$ wird die organische Phase eingedampft, der Rückstand in Ether gelöst und das Produkt durch Einleiten von HCl als Hydrochlorid ausgefällt. Ausbeute: 79 % Fp.: 250°C

In analoger Weise wurden hergestellt:

Beispiel 2

5-Amino-2-methoxy-5,6,7,8-tetrahydro-naphthalin-hydrochlorid

Ausbeute: 79 % der Theorie; Fp.: 262°C.

Beispiel 3

6-Amino-1-methoxy-5,6,7,8-tetrahydro-naphthalin-hydrochlorid

Ausbeute: 62 % der Theorie; Fp.: 258°C.

Beispiel 4

6-Amino-2-methoxy-5,6,7,8-tetrahydro-naphthalin-hydrochlorid

Ausbeute: 42 % der Theorie; Fp.: 239°C.

Beispiel 5

7-Amino-1-methoxy-5,6,7,8-tetrahydro-naphthalin-hydrochlorid

Ausbeute: 61,4 % der Theorie; Fp.: 97°C.

Beispiel 6

5-Amino-1-hydroxy-5,6,7,8-tetrahydro-naphthalin-hydrobromid

0,15 Mol 5-Amino-1-methoxy-5,6,7,8-tetrahydro-naphthalin-hydrochlorid werden in 75 ml wäßriger 48 %iger HBr 3 h auf 125°C Badtemperatur erhitzt. Es wird eingedampft, der Rückstand in wenig Ethanol gelöst und die Produkte durch Zugabe von Ether als Hydrobromide ausgefällt.
Ausbeute: 80 % der Theorie; Fp.: 152°C.
In analoger Weise wurden dargestellt:

Beispiel 7

5-Amino-2-hydroxy-5,6,7,8-tetrahydro-naphthalin-hydrobromid

Ausbeute: 18 % der Theorie

Beispiel 8

6-Amino-1-hydroxy-5,6,7,8-tetrahydro-naphthalin-hydrobromid

Ausbeute: 72 % der Theorie; Fp.: 248°C.

Beispiel 9

6-Amino-2-hydroxy-5,6,7,8-tetrahydro-naphthalin-hydrobromid

Ausbeute: 84 % der Theorie; Fp.: 275°C.

Beispiel 10

7-Benzolsulfonylamino-1-methoxy-5,6,7,8-tetra-hydronaphthalin

10,25 g (56 mmol) 7-amino-1-methoxy-5,6,7,8-tetra-hydronaphthalin werden in 100 ml Pyridin p. a. gelöst, bei RT werden 10,6 g (60 mmol)Benzolsulfonsäurechlorid zugetropft, exotherme Reaktion, 1 h bei RT nachrühren, Reaktionsgemisch i. V. eingeengt, Rückstand über Kieselgelsäule ($K_{60}$, Laufmittel Toluol:Aceton 10:1) gereinigt.

Ausbeute: 10,8 g (61 % der Theorie)
Rf-Wert ($K_{60}$-Folie) 0,75 (Laufmittel: Toluol:Ethanol:Triethylamin 10:3:1).

Beispiel 11

5-Benzoylamino-1-hydroxy-5,6,7,8-tetrahydro-naphthalin

50 mmol Benzoesäure und 50 mmol 1-Hydroxy-benzotriazol (HOBT) werden bei 0°C in 200 ml abs. THF vorgelegt. Dazu werden 55 mmol Dicyclohexylcarbodiimid (DCC) unter $N_2$ gegeben und 1 h bei 0°C, dann 1 h bei RT gerührt. Zu dieser Lösung werden 50 mmol 5-Amino-1-hydroxy-5,6,7,8-tetrahydro-naphthalin-hydrobromid und 50 mmol Triethylamin gegeben und 6 h bei RT gerührt.

Es wird abfiltriert, gut mit THF nachgewaschen und eingedampft. Der Rückstand wird in Essigester aufgenommen und 1 × mit ges. $NaHCO_3$-Lösung, 1 × 1n mit HCl, 1 × mit ges. $NAHCO_3$-Lösung und 1 × mit ges. NaCl-Lösung gewaschen. Nach Trocknen über $Na_2SO_4$ wird eingedampft, in wenig Acetonitril gelöst und nach 1 h im Kühlschrank vom ausgefallenen, restlichen Dicyclohexylharnstoff abfiltriert. Es wird eingedampft und aus Aceton durch Zugabe von Petrolether umkristallisiert.

Ausbeute: 53 % der Theorie; Fp.: 160°C.

In analoger Weise wurden die in Tabelle 1 zusammengefaßten Verbindungen dargestellt:

**Tabelle 1**

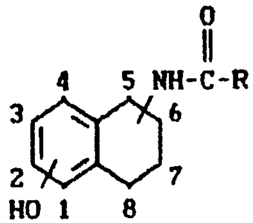

| Beispiel | Substitutionsorte OH | Substitutionsorte NH-CO-R | Rest R | Ausbeute % (in % der Theorie) | Fp. (bei Kristellen) bzw. Wellenzahl der Amidbande im IR-Spektrum bei Ölen |
|---|---|---|---|---|---|
| 12 | 1 | 5 | 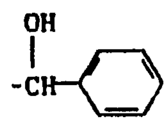 | 76 | 1655 $cm^{-1}$ |
| 13 | 1 | 5 | 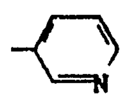 | 83 | $230^0$ C |
| 14 | 2 | 5 | 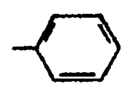 | 31 | 1660 $cm^{-1}$ |
| 15 | 2 | 5 | 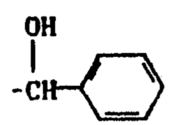 | 27 | 1650 $cm^{-1}$ |

Tabelle 1 (Fortsetzung)

| Beispiel | Substitutionsorte OH | Substitutionsorte NH-CO-R | Rest R | Ausbeute % (in % der Theorie) | Fp. (bei Kristellen) bzw. Wellenzahl der Amidbande im IR-Spektrum bei Ölen |
|---|---|---|---|---|---|
| 16 | 1 | 6 | 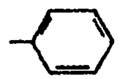 | 60 | 195° C |
| 17 | 1 | 6 | 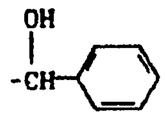 | 77 | 1660 $cm^{-1}$ |
| 18 | 1 | 6 | 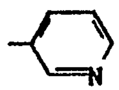 | 58 | 232-33° C |
| 19 | 2 | 6 | 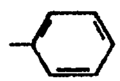 | 82 | 173-75° C |
| 20 | 2 | 6 | 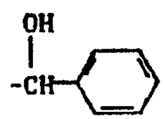 | 58 | 1640 $cm^{-1}$ |
| 21 | 2 | 6 | 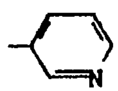 | 85 | 230° C |

Beispiel 22

5-Acetylamino-5,6,7,8-tetrahydro-naphthalin

50 mmol 5-Amino-1-hydroxy-5,6,7,8-tetrahydro-naphthalin werden 2 h in einer Lösung von 200 mmol Acetanhydrid und 200 mmol Triethylamin, der eine Spatelspitze Dimethylaminopyridin zugesetzt ist, gerührt. Es wird abfiltriert, mit THF gut nachgewaschen und eingedampft. Der Rückstand wird in Essigester aufgenommen, mit in HCl, ges. $NaHCO_3$, in HCl, ges. $NaHCO_3$ und ges. NaCl-Lösung gewaschen, über $Na_2SO_4$ getrocknet und eingedampft. Das zurückbleibende Bisacetat wird in 100 ml MeOH gelöst und dann zu 100 ml in KOH bei RT getropft. Es wird 2 h bei 50°C Innentemperatur gerührt, mit konz. HCl angesäuert und das MeOH abgezogen. Die saure wäßrige Phase wird 2 bis 3 × mit Essigester ausgeschüttelt, die vereinigten organischen Phasen werden mit $Na_2SO_4$ getrocknet und eingedampft. Der Rückstand wird aus Essigester umkristallisiert

Ausbeute: 92 % der Theorie $^1$H-NMR ($CD_3OD$): $\delta$ = 1,95 (s, 3H

In analoger Weise wurden dargestellt:

Beispiel 23

5-Acetylamino-2-hydroxy-5,6,7,8-tetrahydro-naphthalin

Ausbeute: 33 % der Theorie $^1$H-NMR ($CD_3OD$): $\delta$ = 2,0 (s, 3H)

Beispiel 24

6-Acetylamino-1-hydroxy-5,6,7,8-tetrahydro-naphthalin

Ausbeute: 81 % der Theorie $^1$H-NMR ($CD_3OD$): $\delta$ = 1,95 (s, 3H)

Beispiel 25

6-Acetylamino-2-hydroxy-5,6,7,8-tetrahydro-naphthalin

Ausbeute: 82 % der Theorie $^1$H-NMR ($CD_3OD$): $\delta$ = 2,0 (s, 3H)

Beispiel 26

5-Benzoylamino-5,6,7,8-tetrahydro-naphth-1-oxyessigsäuremethylester

50 mmol 5-Benzoylamino-1-hydroxy-5,6,7,8-tetrahydro-naphthalin und 100 mmol wasserfreies Kaliumcarbonat werden in 100 ml abs. DMF 20 Min. bei 50°C gerührt. Dazu wird eine Lösung von 60 mmol Chloressigsäuremethylester und 25 mmol Kaliumiodid in 25 ml abs. DMF getropft und 6 bis 8 h bei 50°C gerührt. Es wird warm abfiltriert, gut mit Essigester nachgewaschen und eingedampft. Der Rückstand wird 10 min. in einem Gemisch von 10 % NaOH und Essigester oder Tetrahydrofuran verrührt. Die Essigesterphase wird 1 × mit 10 % NaOH ausgeschüttelt, über $Na_2SO_4$ getrocknet und eingedampft. Der Rückstand wird aus Essigester umkristallisiert.

Ausbeute: 86 % der Theorie; Fp.: 242°C

In analoger Weise wurden die Beispiele der Tabelle 2 hergestellt:

**Tabelle 2**

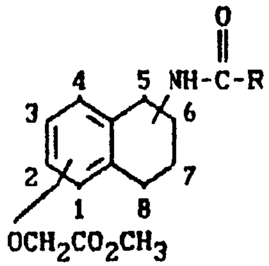

| Beispiel Nr. | Substitutionsorte $OCH_2CO_2CH_3$ | NH-CO-R | Rest R | Ausbeute % (in % der Theorie) | Fp. bei Kristallen bzw. Wellenzahl der Amidbande im IR-Spektrum bei Ölen |
|---|---|---|---|---|---|
| 27 | 1 | 5 | $-CH_3$ | 92 | 196° C |
| 28 | 1 | 5 | 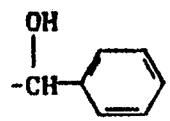 | 72 | 115° C |
| 29 | 1 | 5 | 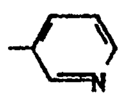 | 52 | 1665 $cm^{-1}$ |
| 30 | 2 | 5 | $-CH_3$ | 33 | 165° C |
| 31 | 2 | 5 | 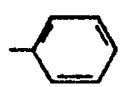 | 91 | 149-150° C |

<u>Tabelle 2</u> (Fortsetzung)

| Beispiel Nr. | Substitutionsorte $OCH_2CO_2CH_3$ | NH-CO-R | Rest R | Ausbeute % (in % der Theorie) | Fp. bei Kristallen bzw. Wellenzahl der Amidbande im IR-Spektrum bei Ölen |
|---|---|---|---|---|---|
| 32 | 2 | 5 | 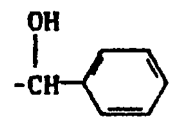 | 45 | 1665 $cm^{-1}$ |
| 33 | 1 | 6 | $-CH_3$ | 81 | 120° C |
| 34 | 1 | 6 | 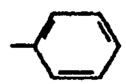 | 69 | 125-27° C |
| 35 | 1 | 6 | 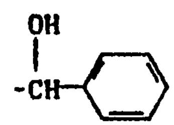 | 21 | 1670 $cm^{-1}$ |
| 36 | 1 | 6 | 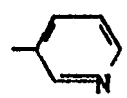 | 75 | 1665 $cm^{-1}$ |

Tabelle 2 (Fortsetzung)

| Beispiel Nr. | Substitutionsorte $OCH_2CO_2CH_3$ | NH-CO-R | Rest R | Ausbeute % (in % der Theorie) | Fp. bei Kristallen bzw. Wellenzahl der Amidbande im IR-Spektrum bei Ölen |
|---|---|---|---|---|---|
| 37 | 2 | 6 | $-CH_3$ | 82 | 110-113°C |
| 38 | 2 | 6 | 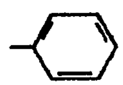 | 47 | 130°C |
| 39 | 2 | 6 | 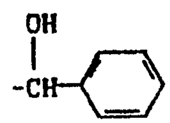 | 14 | 1665 $cm^{-1}$ |
| 40 | 2 | 6 | 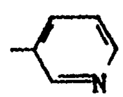 | 44 | 1660 $cm^{-1}$ |

Beispiel 41

6-Amino-5,6,7,8-tetrahydro-naphth-1-oxyessigsäure

20 mmol 6-Acetylamino-5,6,7,8-tetrahydro-naphth-1-yl-oxyessigsäuremethylester werden in 300 ml 2n HCl 24 h refluiert. Beim Abkühlen auf 0°C fällt das Produkt aus.
Ausbeute: 89 % der Theorie; Fp.: 271-73°C
In analoger Weise wurden dargestellt:

Beispiel 42

5-Amino-5,6,7,8-tetrahydro-naphth-1-oxyessigsäure

Ausbeute: 17 % der Theorie $^1$H-NMR ($CD_3OD$): $\delta$ = 4,65 (s, 2H)

Beispiel 43

5-Amino-5,6,7,8-tetrahydro-naphth-2-yl-oxyessigsäure

Ausbeute: 14 % der Theorie $^1$H-NMR ($CD_3OD$): $\delta$ = 4,65 (s, 2H)

Beispiel 44

6-Amino-5,6,7,8-tetrahydro-naphth-2-yl-oxyessigsäure

Ausbeute: 71 % der Theorie; Fp.: 305°C

Beispiel 45

7,8-Tetrahydro-5(6H)-naphthalenon-1-yl-oxyessigsäuremethylester

0, 5 Mol 5-Hydroxy-1-tetralon werden zusammen mit 0,6 Mol Chloressigsäuremethylester, 0,25 Mol Kaliumiodid und 1 Mol Kaliumcarbonat in 1 l Butanon 6 h refluiert. Es wird abgesaugt, gut mit Aceton nachgewaschen und eingedampft. Der Rückstand wird in 500 ml Methylenchlorid aufgenommen, dreimal mit 0,5 NaOH und einmal mit Wasser gewaschen, über Natriumsulfat getrocknet und eingedampft. Der Rückstand wird am Kugelrohr destilliert. Sdp.: 250°C/O, 3 mm
Ausbeute: 83 % der Theorie nach Umkristallisation aus Ligroin Fp: 84 - 85 °C.
In analoger Weise wurde dargestellt:

Beispiel 46

7,8-Tetrahydro-5(6H)-anaphthalenon-2-yl-oxyessigsäuremethylester

Ausbeute: 76 % der Theorie; Fp.: 116-118°C

Beispiel 47

5-Amino-5,6,7,8-tetrahydro-naphth-1-yl-oxyessigsäureamid

50 mmol 7,8-Tetrahydro-5(6H)-naphthalenon-1-yl-oxyessigsäuremethylester werden in 100 ml Methanol und 50 ml $NH_3$ 5 h bei 110°C und 100 bar Wasserstoff über 5 g Raney-Nickel umgesetzt. Es wird heiß abgesaugt, eingedampft und der Rückstand aus Toluol umkristallisiert.
Ausbeute: 68 % Fp.: 133-140°C

Beispiel 48

5-Amino-5,6,7,8-tetrahydro-naphth-2-yl-oxyessigsäureamid

Ausbeute: 89 % der Theorie $^1$H-NMR ($CD_3OD$): $\delta$ = 4,45 (s, 2H)

Beispiel 49

5-(4-Chlor-benzolsulfonylamino)-5,6,7,8-tetrahydro-naphth-2-yl-oxyessigsäureamid

Zu 30 mmol 5-Amino-5,6,7,8-tetrahydro-naphth-2-yl-oxyessigsäureamid und 60 mmol Triethylamin in

80 ml abs. THF werden bei Raumtemperatur 33 mmol p-Chlorsulfonsäurechlorid in 20 ml abs. THF zugetropft. Es wird 6 h bei RT nachgerührt, abfiltriert und 30 ml Ether zugesetzt. Die organische Phase wird mit 1n HCl, ges. $NaHCO_3$-Lsg. und ges. NaCl-Lsg. gewaschen, über $Na_2SO_4$ getrocknet und eingedampft.

Ausbeute 58 % der Theorie; $^1H$-NMR($d_6$-DMSO): δ = 4,35 (s, 2H); Fp.: 203-5°C

Beispiel 50

5-(4-Chlor-benzolsulfonylamino)-5,6,7,8-tetrahydro-naphth-1-yl-oxyessigsäureamid

Ausbeute: 52 % der Theorie; Fp.: 205-208°C $^1H$-NMR ($d_6$-DMSO): δ 4,45 (s, 2H)

Beispiel 51

7-Benzolsulfonylamino-5,6,7,8-tetrahydro-1-hydroxynaphthalin

9,6 g (30 mmol) 7-Benzolsulfonylamino-1-methoxy-5,6,7,8-tetrahydronaphthalin werden in 150 ml $CH_2Cl_2$ p. a. gelöst, bei RT werden 40 ml 1molare (40 mmol) Bortribromid-Lsg. zugetropft, 1 h bei RT nachgerührt, Reaktionsgemisch auf Eiswasser, das etwas L-(+)-Weinsäure enthält gegeben, $CH_2Cl_2$ abgetrennt, über $MgSO_4$ getrocknet, i. V. eingeengt, Rückstand kristallin.

Ausbeute: 5,7 g (62,7 % der Theorie)

Fp: 152-154°C

Beispiel 52

7-Benzolsulfonylamino-5,6,7,8-tetrahydronaphth-1-yl-oxyessigsäureethylester

5,3 g (17,5 mmol) 7-Benzolsulfonylamino-5,6,7,8-tetrahydro-1-hydroxy-naphthalin und 2,76 g (20 mmol) gemahlenes $K_2CO_3$ werden in 100 ml DMF p.a. gerührt, 3,35 g (20 mmol) Bromessigsäureethylester werden bei RT zugetropft, 1 h bei RT nachgerührt, Reaktionsgemisch filtriert, Mutterlauge i. V. eingeengt.

Ausbeute: 6,6 g (97 % der Theorie)

Rf-Wert: $K_{60}$-Folie 0,45 (Laufmittel Toluol: Aceton 4:1)

Beispiel 53

7-Benzolsulfonylamino-5,6,7,8-tetrahydronaphth-1-yl-oxyessigsäure

3,89 (10 mmol) 7-Benzolsulfonylamino-5,6,7,8-tetrahydronaphth-1-yl-oxyessigsäureethylester und 840 mg (15 mmol) KOH werden in 50 ml $C_2H_5OH$ und 20 ml $H_2O$ 20 h bei RT gerührt. Das Lösungsmittel wird abgezogen und der Rückstand zwischen $H_2O$ und $CH_2Cl_2$ verteilt, mit 10 %iger HCl auf pH 4,5 eingestellt, 2 × mit $CH_2Cl_2$ extrahiert, organische Phasen über $MgSO_4$ getrocknet, i. V. eingeengt, Rückstand mit Ether verrieben, Kristalle abgesaugt, getrocknet.

Ausbeute: 1,8 g (49,7 % der Theorie)

$F_p$: 205°C

Beispiel 54

6-Benzolsulfonylamino-5,6,7,8-tetrahydro-naphth-1-yl-oxyessigsäure

10 mmol 6-Amino-5,6,7,8-tetrahydro-naphth-1-yl-oxyessigsäure und 20 mmol Benzolsulfonylsäurechlorid werden 2 h in 40 ml 10 %iger NaOH auf 80°C erwärmt. Dann wird mit konz. HCl angesäuert und die milchige Suspension 2 × mit EE ausgeschüttelt. Die vereinigten Essigesterphasen werden schnell mit 10 % NaOH ausgeschüttelt (Produkt fällt aus Essigester aus), die NaOH Phase wird mit konz. HCl angesäuert, 2 × mit Essigester ausgeschüttelt, über $Na_2SO_4$ getrocknet und eingedampft. Der Rückstand wird in Essigester aufgekocht und filtriert. Das Produkt kristallisiert nach 2-3 h im Kühlschrank aus.

Ausbeute 63 % der Theorie $^1H$-NMR(NaOD): δ = 4,55 (s, 2H)

Fp: 175°C

Analog wurden hergestellt:

Beispiel 55

6-(4-Chlorbenzolsulfonylamino)-5,6,7,8-tetrahydro-naphth-1-yl-oxyessigsäure

Ausbeute: 67 % der Theorie; Fp.: 248°C

Beispiel 56

6-Benzolsulfonylamino-5,6,7,8-tetrahydro-naphth-2-yl-oxyessigsäure
Ausbeute: 54 % der Theorie $^1$H-NMR (NaOD): δ 4,5 (s, 2H)

Beispiel 57

5-(4-Chlorbenzolsulfonylamino)-5,6,7,8-tetrahydro-naphth-2-yl-oxyessigsäure

10 mmol 5-(4-Chlorbenzolsulfonylamino)-5,6,7,8-tetrahydro-naphth-2-yl-oxyessigsäureamid werden in 12 ml KOH und 40 ml MeOH 6 h zum Rückfluß erhitzt. Das Methanol wird abgezogen, es werden 30 ml in KOH zugesetzt und 2 × mit $CH_2Cl_2$ ausgeschüttelt. Die $H_2O$ Phase wird mit konz. HCl auf pH 2 gebracht, der Niederschlag wird abfiltriert und gut getrocknet.
Ausbeute : 71 % der Theorie
$^1$H-NMR ($d_6$-DMSO): δ = 4,6 (s, 2H)
Analog wurde hergestellt:

Beispiel 58

5-(4-Chlorbenzolsulfonylamino)-5,6,7,8-tetrahydro-naphth-1-yl-oxyessigsäure

Ausbeute: 66 % der Theorie; Fp.: 210°C (Essigester/Petrolether)

Beispiel 59

5-(4-Fluorbenzolsulfonylamino)-5,6,7,8-tetrahydro-naphth-1-yl-oxyessigsäure

Ausbeute: 68 % der Theorie; Fp.:

Beispiel 60

5-Benzoylamino-5,6,7,8-tetrahydro-naphth-1-oxyessigsäure

50 mmol 5-Benzoylamino-5,6,7,8-tetrahydro-naphth-1-yl-oxyessigsäuremethylester werden in 60 ml in NaOH und 120 ml MeOH 6 h bei Raumtemperatur gerührt. Das Methanol wird abgezogen, die NaOH-Lösung mit Essigester ausgeschüttelt und dann mit 10 % HCl Lösung angesäuert. Die saure Lösung wird 3 × mit Essigester oder THF ausgeschüttelt, über $Na_2SO_4$ getrocknet, eingedampft und am Hochvakuum getrocknet
Ausbeute: 94 % der Theorie; IR: 1745 $cm^{-1}$ ($CO_2H$)
Weitere Beispiele, die in analoger Weise hergestellt wurden, sind in Tabelle 3 zusammengestellt:

**Tabelle 3**

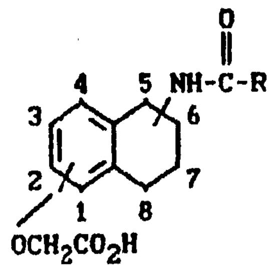

| Beispiel Nr. | Substitutionsorte $OCH_2CO_2H$ | Substitutionsorte NH-CO-R | Rest R | Ausbeute % (in % der Theorie) | Wellenzahl der Carbonylbande im IR-Spektrum |
|---|---|---|---|---|---|
| 61 | 1 | 5 | 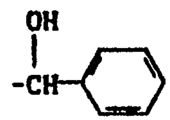 | 91 | 1735 $cm^{-1}$ |
| 62 | 1 | 5 | 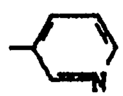 | 87 | 1730 $cm^{-1}$ |
| 63 | 2 | 5 | 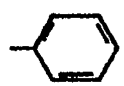 | 90 | 1755 $cm^{-1}$ |
| 64 | 2 | 5 | 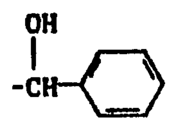 | 92 | 1725 $cm^{-1}$ |

**Tabelle 3** (Fortsetzung)

| Beispiel Nr. | Substitutionsorte $OCH_2CO_2H$ | NH-CO-R | Rest R | Ausbeute % (in % der Theorie) | Wellenzahl der Amidbande im IR-Spektrum bei Ölen |
|---|---|---|---|---|---|
| 65 | 1 | 6 | 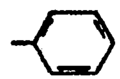 | 96 | 1745 $cm^{-1}$ |
| 66 | 1 | 6 | 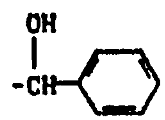 | 94 | 1740 $cm^{-1}$ |
| 67 | 1 | 6 | 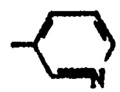 | 89 | 1730 $cm^{-1}$ |
| 68 | 2 | 6 | 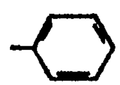 | 95 | 1725 $cm^{-1}$ |
| 69 | 2 | 6 | 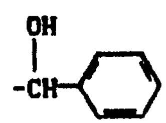 | 95 | 1725 $cm^{-1}$ |
| 70 | 2 | 6 | 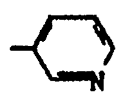 | 89 | 1725 $cm^{-1}$ Fp. 211°C |

## Patentansprüche

1. Amino-5,6,7,8-tetrahydronaphthyl-oxyessigsäuren der allgemeinen Formel (I)

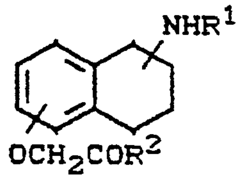

(I)

in welcher
$R^1$ für

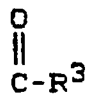

oder $SO_2R^4$ steht, wobei

$R^3$ für Aryl, substituiertes Aryl, Heteroaryl, Aralkyl oder die Gruppe

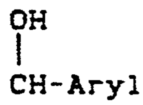

steht und wobei

$R^4$ für Aryl oder substituiertes Aryl steht,

$R^2$ für OH, Alkoxy, Phenoxy, Benzoxy oder $NR^5R^6$ steht wobei

$R^5$ und $R^6$ gleich oder verschieden sind und jeweils für Wasserstoff oder Alkyl stehen oder einer der Reste $R^5$ oder $R^6$ für Benzyl steht,
sowie deren physiologisch verträglichen Salze mit ein-oder zweiwertigen Kationen.

2. Verbindungen der allgemeinen Formel (I) gemäß Anspruch 1 in welcher

$R^1$ für $CO\text{-}R^3$ oder $SO_2R^4$ steht, wobei $R^3$ für Phenyl oder Naphthyl steht, welches gegebenenfalls 1, 2 oder 3 gleiche oder verschiedene Substituenten aus der Gruppe Halogen, Cyano, Trifluormethyl und Alkyl mit 1 bis 4 C-Atomen trägt oder für Pyridin, Chinolin oder Aralkyl mit 7 bis 12 Kohlenstoffatomen steht, wobei der Aralkylrest gegebenenfalls im Alkylteil durch Halogen oder Hydroxy substituiert ist und gegebenenfalls im Arylteil durch Halogen oder Alkyl mit 1 bis 4 Kohlenstoffatomen substituiert ist
oder für die Gruppe CHOH-Aryl steht wobei Aryl Phenyl oder Naphthyl bedeutet, welches gegebenenfalls 1 bis 3-fach substituiert ist durch Halogen, Cyano, Trifluormethyl oder Alkyl mit bis 4 Kohlenstoffatomen,
$R^4$ für Phenyl oder Naphthyl steht, welche gegebenenfalls 1, 2 oder 3 gleiche oder verschiedene Substituenten aus der Gruppe Halogen, Cyano, Trifluormethyl und Alkyl mit 1 bis 4 C-Atomen tragen,

$R^2$ für Hydroxy, Phenoxy, Benzoxy oder Alkoxy mit 1 bis 4 Kohlenstoffatomen steht oder für die Gruppe $NR^5R^6$ steht, wobei $R^5$ und $R^6$ gleich oder verschieden sind und jeweils für Wasserstoff oder Alkyl mit 1 bis 4 Kohlenstoffatomen stehen oder einer der Reste $R^5$ oder $R^6$ für Benzyl steht,

sowie deren physiologisch verträglichen Salze mit ein- oder zweiwertigen Kationen.

3. Verbindungen der allgemeinen Formel (I) gemäß Anspruch 1, in welcher in den Substituentendefinitionen Halogen für Fluor oder Chlor steht.

4. Verfahren zur Herstellung von Amino-5,6,7,8-tetrahydronaphthyl-oxyessigsäuren der allgemeinen Formel (I)

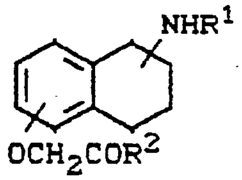

(I)

in welcher

$R^1$ für

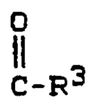

oder $SO_2R^4$ steht, wobei

$R^3$für Aryl, substituiertes Atyl, Heteroaryl, Aralkyl oder die Gruppe

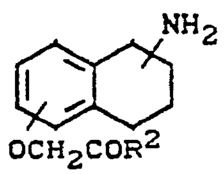

(II)

Aryl steht und wobei

$R^4$für Aryl oder substituiertes Aryl steht,

$R^2$für OH, Alkoxy, Phenoxy, Benzoxy oder $NR^5R^6$ steht wobei

$R^5$ und $R^6$gleich oder verschieden sind und jeweils für Wasserstoff oder Alkyl stehen oder einer der Reste $R^5$ oder $R^6$ für Benzyl steht,

dadurch gekennzeichnet, daß man Amine der allgemeinen Formel (II)

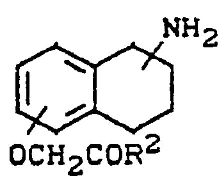

(II)

in welcher $R^2$ die oben angegebene Bedeutung hat,

mit Säuren der allgemeinen Formeln $R^3$-COOH oder $R^4SO_3H$

worin $R^3$ und $R^4$ die oben angegebene Bedeutung besitzen

oder mit deren aktivierten Derivaten wie Säurechloriden, -anhydriden oder aktivierten Estern umsetzt, wobei im Falle für $R^2$ OH gegebenenfalls eine Verseifung zur freien Carbonsäure durchgeführt wird, oder

daß man Phenole der allgemeinen Formel (III)

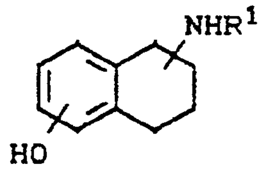

(III)

in welcher $R^1$ die oben angegebene Bedeutung hat,

mit Essigsäurederivaten der allgemeinen Formel (IV)

$X\text{-}CH_2\text{-}COR^2$ (IV)

in welcher

Xeine Abgangsgruppe wie Chlor, Brom, Jod, $SO_2CH_3$ oder Tosyl bedeutet und
$R^2$die oben angegebene Bedeutung besitzt,

in Gegenwart von säurebindenden Mitteln umsetzt, wobei für den Fall, daß $R^2$ OH bedeutet, gegebenenfalls eine Verseifung zur freien Carbonsäure erfolgt.

5. Verfahren zur Herstellung von Verbindungen der allgemeinen Formel (II) gemäß Anspruch4, dadurch gekennzeichnet, daß man Hydroxytetraline der allgemeinen Formel (VI)

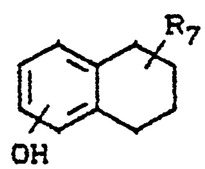

(VI)

in welcher $R_7$ eine Ketogruppe bedeutet,

mit Essigsäurederivaten der allgemeinen Formel (IV)

$X\text{-}CH_2\text{-}COR^2$(IV)

in welcher X und $R^2$ die in Anspruch 4 angegebene Bedeutung haben,

alkyliert und die erhaltenen Tetralone der allgemeinen Formel (V)

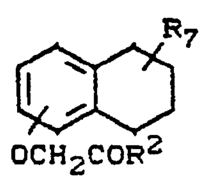

(V)

in welcher $R^2$ und $R^7$ die oben angegebene Bedeutung haben,

reduktiv aminiert,

oder

Acetamide der allgemeinen Formel (VII)

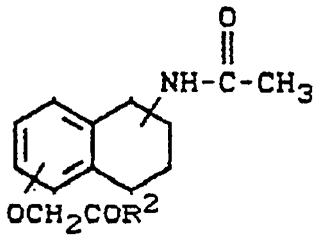

(VII)

in welcher $R^2$ die oben angegebene Bedeutung hat,

sauer oder basisch hydrolysiert.

6. Verbindungen der allgemeinen Formel (II)

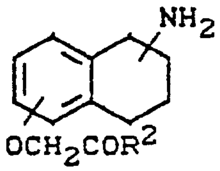

(II)

in welcher $R^2$ die in Anspruch 1 angegebene Bedeutung hat.

7. Verwendung von Verbindungen der allgemeinen Formel (II) gemäß Anspruch 6 zur Herstellung von Verbindungen der allgemeinen Formel (I) gemäß Anspruch 1.

8. Arzneimittel enthaltend eine Verbindung der allgemeinen Formel (I) gemäß Anspruch 1.

9. Verfahren zur Herstellung von Arzneimitteln, dadurch gekennzeichnet, daß man Verbindungen der allgemeinen Formel (I) gemäß Anspruch 1, gegebenenfalls unter Verwendung von inerten Hilfs- und Trägerstoffen in eine geeignete Applikationsform überführt.

10. Verwendung von Verbindungen der allgemeinen Formel (I) gemäß Anspruch 1 bei der Herstellung von Arzneimitteln zur Bekämpfung von thrombo-embolischen, ischämischen und arteriosklerotischen Erkrankungen.

**Claims**

1. Amino-5,6,7,8-tetrahydronaphthyl-oxyacetic acids of the general formula (I)

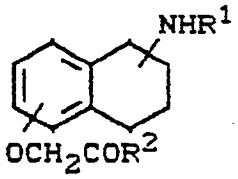

(I)

in which
$R^1$ represents

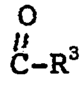

or $SO_2R^4$ wherein $R^3$ represents aryl, substituted aryl, heteroaryl, aralkyl or the group

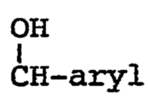

and wherein $R^4$ represents aryl or substituted aryl, and $R^2$ represents OH, alkoxy, phenoxy, benzoxy or $NR^5R^6$, wherein $R^5$ and $R^6$ are identical or different and each represents hydrogen or alkyl, or one of the radicals $R^5$ or $R^6$ represents benzyl, and physiologically acceptable salts thereof with monoor divalent cations.

2. Compounds of the general formula (I) according to Claim 1, in which $R^1$ represents CO–$R^3$ or $SO_2R^4$, wherein $R^3$ represents phenyl or naphthyl which optionally carries 1, 2 or 3 identical or different substituents from the group comprising halogen, cyano, trifluoromethyl and alkyl with 1 to 4 C atoms, or represents pyridine, quinoline or aralkyl with 7 to 12 carbon atoms, the aralkyl radical optionally being substituted in the alkyl part by halogen or hydroxyl and optionally being substituted in the aryl part by halogen or alkyl with 1 to 4 carbon atoms, or represents the group CHOH-aryl, wherein aryl denotes phenyl or naphthyl which is optionally substituted by 1, 2 or 3 radicals from the group comprising halogen, cyano, trifluoromethyl and alkyl with 1 to 4 carbon atoms, $R^4$ represents phenyl or naphthyl, which optionally carry 1, 2 or 3 identical or different substituents from the group comprising halogen, cyano, trifluoromethyl and alkyl with 1 to 4 C atoms, and $R^2$ represents hydroxyl, phenoxy, benzoxy or alkoxy with 1 to 4 carbon atoms, or represents the group $NR^5R^6$, wherein $R^5$ and $R^6$ are identical or different and each represent hydrogen or alkyl with 1 to 4 carbon atoms, or one of the radicals $R^3$ or $R^6$ represents benzyl, and physiologically acceptable salts thereof with mono- or divalent cations.

3. Compounds of the general formula (I) according to Claim 1, in which halogen represents fluorine or chlorine in the substituent definitions.

4. Process for the preparation of amino-5,6,7,8-tetrahydronaphthyl-oxyacetic acids of the general formula (I)

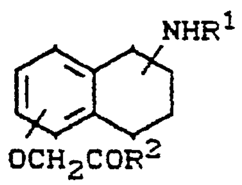

(I)

in which $R^1$ represents

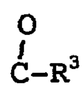

or $SO_2R^4$ wherein $R^3$ represents aryl, substituted aryl, heteroaryl, aralkyl or the group

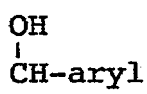

and wherein $R^4$ represents aryl or substituted aryl, and $R^2$ represents OH, alkoxy, phenoxy, benzoxy or $NR^5R^6$, wherein $R^5$ and $R^6$ are identical or different and each represents hydrogen or alkyl, or one of the radicals $R^5$ or $R^6$ represents benzyl, characterized in that amines of the general formula (II)

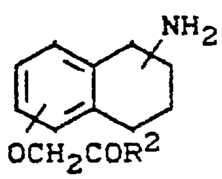

(II)

in which $R^2$ has the abovementioned meaning, are reacted with acids of the general formula $R^3$–COOH or $R^4SO_3H$ wherein $R^3$ and $R^4$ have the abovementioned meaning, or with activated derivatives thereof, such as acid chlorides or anhydrides or activated esters, in the case where $R^2 \neq$ OH, hydrolysis to give the free carboxylic acid being carried out, if appropriate, or in that phenols of the general formula (III)

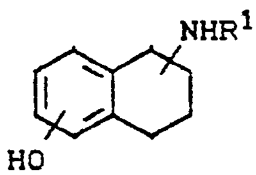

(III)

in which $R^1$ has the abovementioned meaning, are reacted with acetic acid derivatives of the general formula (IV)

X–$CH_2$–$COR^2$ (IV)

in which X denotes a leaving group, such as chlorine, bromine, iodine, $SO_2CH^3$ or tosyl and $R^2$ has the abovementioned meaning, in the presence of acid-binding agents, in the case where $R^2 \neq OH$, hydrolysis to give the free carboxylic acid being carried out, if appropriate.

5. Process for the preparation of compounds of the general formula (II) according to Claim 4, characterized in that hydroxytetralins of the general formula (VI)

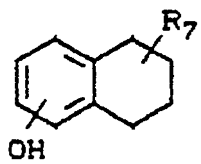

(VI)

in which $R_7$ denotes a keto group, are alkylated with acetic acid derivatives of the general formula (IV)

X–$CH_2$–$COR^2$ (IV)

in which X and $R^2$ have the meaning given in Claim 4, and the resulting tetralones of the general formula (V)

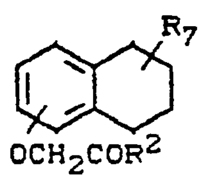

(V)

in which R and $R^7$ have the abovementioned meaning, are subjected to reductive amination, or acetamides of the general formula (VII)

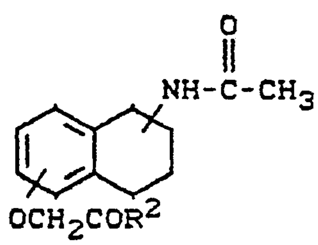

(VII)

in which $R^2$ has the abovementioned meaning, are subjected to acid or basic hydrolysis.

6. Compounds of the general formula (II)

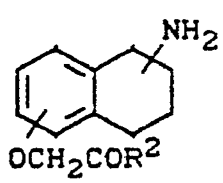

(II)

in which $R^2$ has the meaning given in Claim 1.

7. Use of compounds of the general formula (II) according to Claim 6 for the preparation of compounds of the general formula (I) according to Claim 1.

8. Medicaments containing a compound of the general formula (I) according to Claim 1.

9. Process for the preparation of medicaments, characterized in that compounds of the general formula (I) according to Claim 1 are converted into a suitable administration form, if appropriate using inert auxiliaries and excipients.

10. Use of compounds of the general formula (I) according to Claim 1 in the preparation of medicaments for combating thrombo-embolic, ischaemic and arteriosclerotic diseases.

## Revendications

1. Acides amino-5,6,7,8-tétrahydronaphtyl-oxyacétiques répondant à la formule générale (I)

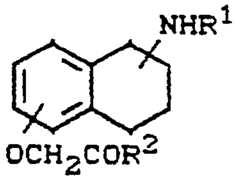

(I)

dans laquelle
$R^1$ représente

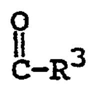

ou $SO_2R^4$,
$R^3$ représentant un groupe aryle, un groupe aryle substitué, un groupe hétéroaryle, un groupe aralkyle ou le groupe

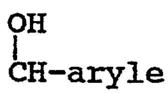 et

$R^4$ représente un groupe aryle ou un groupe aryle substitué,
$R^2$ représente OH, une group alcoxy, un groupe phénoxy, un groupe benzoxy ou $NR^5R^6$, où
$R^5$ et $R^6$ sont identiques ou différents et représentent chacun un atome d'hydrogène ou un groupe alkyle, ou bien un des radicaux $R^5$ ou $R^6$ représente un groupe benzyle, ainsi que leurs sels physiologiquement acceptables, contenant des cations mono- ou bivalents.

2. Composés répondant à la formule générale (I) selon la revendication 1, dans laquelle
$R^1$ représente $CO-R^3$ ou encore $SO_2R^4$, où $R^3$ représente un groupe phényle ou un groupe naphtyle qui porte éventuellement 1, 2 ou 3 substituants identiques ou différents choisis parmi le groupe comprenant un atome d'halogène, un groupe cyano, un groupe trifluorométhyle et un groupe alkyle contenant 1 à 4 atomes de carbone, ou bien un groupe pyridyle, un groupe quinolyle ou un groupe aralkyle contenant 7 à 12 atomes de carbone, le radical aralkyle étant éventuellement substitué par un atome d'halogène ou un groupe hydroxy dans la fraction alkyle et étant éventuellement substitué par un atome d'halogène ou un groupe alkyle contenant 1 à 4 atomes de carbone dans la fraction aryle, ou encore le groupe CHOH-aryle, le groupe aryle représentant un groupe phényle ou un groupe naphtyle qui peut être éventuellement substitué une à trois fois par un atome d'halogène, un groupe cyano, un groupe trifluorométhyle ou un groupe alkyle contenant 1 à 4 atomes de carbone,
$R^4$ représente un groupe phényle ou un groupe naphtyle qui peut porter, éventuellement, 1, 2 ou 3 substituants identiques ou différents choisis parmi le groupe comprenant un atome d'halogène, un groupe cyano, un groupe trifluorométhyle et un groupe alkyle contenant 1 à 4 atomes de carbone,
$R^2$ représente un groupe hydroxyle, un groupe phénoxy, un groupe benzoxy ou un groupe alcoxy contenant 1 à 4 atomes de carbone, ou bien le groupe $NR^5R^6$ où $R^5$ et $R^6$ sont identiques ou différents et représentent, chacun, un atome d'hydrogène ou un groupe alkyle contenant 1 à 4 atomes de carbone, ou bien un des radicaux $R^5$ ou $R^6$ représente un groupe benzyle, ainsi que leurs sels physiologiquement acceptables, contenant des cations mono- ou bivalents.

3. Composés répondant à la formule générale (I) selon la revendication 1, dans laquelle l'atome d'halogène représente un atome de fluor ou un atome de chlore, dans les définitions des substituants.

4. Procédé pour la préparation d'acides amino-5,6,7,8-tétrahydronaphtyl-oxyacétiques répondant à la formule générale (I)

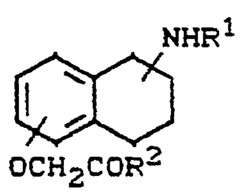

(I)

dans laquelle
$R^1$ représente

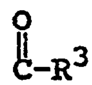

ou $SO_2R_4$,
$R^3$ représentant un groupe aryle, un groupe aryle substitué, un groupe hétéroaryle, un groupe aralkyle ou le groupe

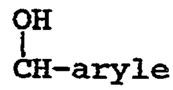 et

$R^4$ représente un groupe aryle ou un groupe aryle substitué,
$R^2$ représente OH, un groupe alcoxy, un groupe phénoxy, un groupe benzoxy ou $NR^5R^6$, où
$R^5$ et $R^6$ sont identiques ou différents et représentent chacun un atome d'hydrogène ou un groupe alkyle, ou bien un des radicaux $R^5$ ou $R^6$ représente un groupe benzyle, caractérisé en ce qu'on fait réagir des amines répondant à la formule générale (II)

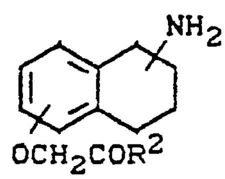

(II)

dans laquelle $R^2$ a la signification définie ci-dessus, avec des acides répondant à la formule générale

$R^3$–COOH ou $R^4SO_3H$

où $R^3$ et $R^4$ ont les significations définies ci-dessus
ou avec des dérivés activés de ces derniers, tels que les chlorures d'acides, les anhydrides d'acides ou les esters activés, une saponification ayant éventuellement lieu pour l'obtention des acides carboxyliques libres, au cas où $R^2$ n'est pas égal à OH, ou bien en ce qu'on fait réagir des phénols répondant à la formule générale (III)

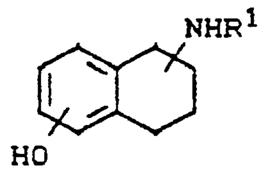

(III)

dans laquelle $R^1$ a la signification indiquée ci-dessus,
avec des dérivés de l'acide acétique répondant à la formule générale (IV)

X–$CH_2$–$COR^2$ (IV)

dans laquelle
X représente un groupe qui se sépare, tel que le chlore, le brome, l'iode, $SO_2CH^3$ ou un groupe tosyle et $R^2$ a la signification définie ci-dessus, en présence d'agents fixateurs d'acides, une saponification ayant éventuellement lieu pour l'obtention des acides carboxyliques libres au cas où $R^2$ est différent de OH.

5. Procédé pour la préparation de composés répondant à la formule générale (II) selon la revendication 4, caractérisé en ce qu'on alkyle des hydroxytétralines répondant à la formule générale (VI)

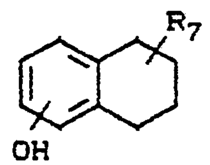

(VI)

dans laquelle $R^7$ représente un groupe céto,
avec des dérivés de l'acide acétique, répondant à la formule générale (IV)

$X-CH_2-COR^2$ (IV)

dans laquelle X et $R^2$ ont la signification définie dans la revendication 4,
et en ce qu'on amine par réduction les tétralones obtenues répondant à la formule générale (V)

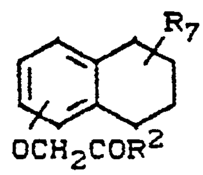

(V)

ou bien on soumet à une hydrolyse acide ou basique des acétamides répondant à la formule générale (VII)

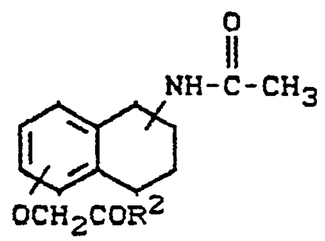

(VII)

dans laquelle $R^2$ a la signification indiquée ci-dessus.

6. Composés répondant à la formule générale (II)

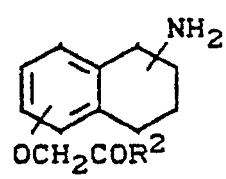

(II)

dans laquelle $R^2$ a la signification définie dans la revendication 1.

7. Utilisation des composés répondant à la formule générale (II) selon la revendication 6, pour la préparation de composés répondant à la formule générale (I) selon la revendication 1.

8. Médicament contenant un composé répondant à la formule générale (I) selon la revendication 1.

9. Procédé pour la préparation de médicaments, caractérisé en ce qu'on transforme des composés répondant à la formule générale (I) selon la revendication 1, en une forme d'application appropriée, en utilisant éventuellement des substances auxiliaires ou de support inertes.

10. Utilisation de composés répondant à la formule générale (I) selon la revendication 1, dans la préparation de médicaments destinés à combattre les maladies thrombo-emboliques, ischémiques et artériosclérotiques.